# EUROPEAN PATENT APPLICATION

(11) **EP 2 510 940 A1**
(43) Date of publication of application: **17.10.2012**
(21) Application number: 10827929.0
(22) Date of filing: 05.11.2010
(51) Int. Cl.: A61K 38/17, A61K 31/713, A61P 29/00, A61P 19/02, A61P 19/04, A61P 37/06, C07K 16/18, C07H 21/04

(54) **USE OF CYR61 PROTEIN FOR PREPARING MEDICINE**

(30) Priority: 06.11.2009 CN 200910198380
(71) Applicant: Shanghai Institute Of Immunology, Luwan, Shanghai 200025 (CN)
(72) Inventor: LI, Ningli, Shanghai 200025 (CN); WANG, Li, Shanghai 200025 (CN); SHEN, Baihua, Shanghai 200025 (CN); XIAO, Lianbo, Shanghai 200025 (CN); OUYANG, Guilin, Shanghai 200025 (CN)
(74) Representative: Lermer, Christoph
(86) International application number: PCT/CN2010/078481
(87) International publication number: WO 2011/054315

(57) **Abstract**

The present invention provides the use of CYR61 protein and inhibitors thereof for preparing medicine for treating rheumatic diseases, wherein the inhibitors are selected from anti-CYR61 antibody or interfering RNA of CYR61 gene. The technical scheme of present invention are: application of CUR61 protein in preparing medicine for treating rheumatic diseases; the rheumatic disease displayed here is rheumatoid arthritis (RA). The advantages of present invention are: discovering the usage of CYR61 as medicine; developing a new field for CYR61 application. Present invention displays the results that CYR61 has ability to enhance proliferation of fibroblast-like synoviocyte (FLS) derived from RA patients, which further results in damage of joint cartilage and bone in RA patients. CYR61 can inhibit FLS apoptosis while increases proliferation of FLS. Another way is that CYR61 induces expression of metal proteases up-regulation which further leads to damage of joint cartilage and bone. The invention also shows using some inhibitors, such as anti-CYR61 antibody or interfering RNA of CYR61 gene can block pathogenesis of CYR61. These results supply experimental evidences for screening therapeutic targets for biomedical and small molecules treatment in the future. The invention has the value for clinical application.

SEQUENCE LISTING

<110>  ÉÏº£ÊÐÃâÒßÑ§ÑÐ¾¿Ëù

<120>  CYR61µ°°×ÔÚÖÆÒ©ÖÐµÄÓ¦ÓÃ

<130>  memo

<150>  CN200910198380.3
<151>  2009-11-06

<160>  10

<170>  PatentIn version 3.3

<210>  1
<211>  20
<212>  DNA
<213>  Artificial

<220>
<223>  GAPDHÒýÎï

<400>  1
acccactcct ccacctttga                                                   20


<210>  2
<211>  22
<212>  DNA
<213>  Artificial

<220>
<223>  GAPDHÒýÎï

<400>  2
ctgttgctgt agccaaattc gt                                                22


<210>  3
<211>  22
<212>  DNA
<213>  Artificial

<220>
<223>  Cyr61ÒýÎï

<400>  3
tccagcccaa ctgtaaacat ca                                                22


<210>  4
<211>  20
<212>  DNA
<213>  Artificial

<220>
<223>  Cyr61ÒýÎï

<400>  4
ggacacagag gaatgcagcc                                                   20


<210>  5
<211>  21

<212>  RNA
<213>  Artificial

<220>
<223>  S1 siRNAÕýÒåÁ´

<220>
<221>  misc_feature
<222>  (20)..(21)
<223>  n=t

<400>  5
ccagaaaugu auuguucaan n                                                21

<210>  6
<211>  21
<212>  RNA
<213>  Artificial

<220>
<223>  S1 siRNA•´ÒåÁ´

<400>  6
uugaacaaua cauuucuggc c                                                21

<210>  7
<211>  21
<212>  RNA
<213>  Artificial

<220>
<223>  S2 siRNAÕýÒåÁ´

<220>
<221>  misc_feature
<222>  (20)..(21)
<223>  n=t

<400>  7
ucaagguauc aauguuuaan n                                                21

<210>  8
<211>  21
<212>  RNA
<213>  Artificial

<220>
<223>  S2 siRNA•´ÒåÁ´

<220>
<221>  misc_feature
<222>  (20)..(20)
<223>  n=t

<400>  8
uugcucgcaa caaaucugcn c                                                21

<210>  9

<211>  21
<212>  RNA
<213>  Artificial

<220>
<223>  S3 siRNAÕýÒåÁ´

<220>
<221>  misc_feature
<222>  (20)..(21)
<223>  n=t

<400>  9
caacgaggac ugcagcaaan n                                                21

<210>  10
<211>  21
<212>  RNA
<213>  Artificial

<220>
<223>  S3 siRNA•´ÒåÁ´

<400>  10
uuugcugcag uccucguuga g                                                21

## Description

### [Technical Field]

This invention is related to application of the protein CYR61, especially usage in pharmacy.

### [Technical Background]

Rheumatoid arthritis (RA), is one of the most common rheumatic diseases. Morbidity of RA in different countries and regions varies slightly and the global average is 0.5~1%. RA is not a benign disease, and compared to contemporaries, lifetime of RA patients may shorten 10~15 years. Meanwhile, disability rate of RA is highest in arthritis. Morbidity of untreated RA patients after 2 and 3 years is 50% and 70%, respectively. RA is a kind of general chronic autoimmune diseases, characterized by synovial inflammation in joints. The clinical feature of RA is aggressive synovial inflammation involved peripheral joints, and is proliferative and destructive like "local malignant tumor". The course of RA is progressive and spontaneous remission is rarely seen. Joints of RA patients in late period could be deformed and disabled. The pathogenesis of RA remains obscure and there is no specific remedy for RA. Since 1990s, combined chemotherapy, imitating treatment of malignant tumor, has launched and became a breakthrough in RA therapy. Nevertheless, drug therapy and operative treatment for RA, such as synovectomy, joint replacement, can only reduce inflammation and relieve pain, and vainly affect on aggressed cartilage and bone. For these reasons, there is momentous theoretical and practical significance to seek a new target to RA treatment.

CYR61 (cysteine-rich 61) is a secreted protein with 42kDa MW, comprising 381 amino acids. The primary cloned CYR61 is discovered by Lau *et al* when they stimulated BALB/c mouse fibroblast 3T3 with serum or platelet derived growth factor (PDGF). It was named after CYR61 for being rich in cysteine (10%). CYR61 has obvious effects on promoting mitosis and chemotaxis and is a basic protein in vital movement, inducing proliferation of fibroblast and secretion of extracellular matrix, regulating cell proliferation, differentiation, embryonic development. At present, researches on CYR61 has focused on tumor while little on RA.

### [Contents of the invention]

This invention aims at providing new application of CYR61 protein.

To this point, the technical proposal adopted by this invention is: application of CYR61 protein in preparation of medicine for rheumatic diseases treatment.

The rheumatic disease referred below is rheumatoid arthritis.

According to above-mentioned technical proposal, detailed enforcement of this invention is as followed:
1. Based on Gene Array, expression profile of CYR61 in different tissues of RA patients was analyzed by Real-time PCR. The expression of CYR61 was high in synovium of RA patients, which was 8.7 times as in periphery blood mononuclear cells, 13.3 times in monocytes of Synovial fluid, and 125 times in synovium of normal persons. Meanwhile, expression of CYR61 was also analyzed in synoviums of RA patients, OA patients and normal persons by immunohistochemistry and the results revealed that CYR61 is expressed highly in synovium of RA patients, compared with OA patients and normal persons. Further, fibroblast-like synoviocytes (FLS) derived from synoviums of RA patients were cultured *in vitro* and researched: CYR61 was expressed highly in cultured FLS than in synovium. Expression profile of CYR61 was compared between cultured FLS derived from RA patients, OA patients and normal persons, confirming that expression of CYR61 was significantly high in FLS of RA patients compared with OA patients and normal persons and suggesting that CYR61 may play an important role in synovium lesions in RA.
2. The relationship between proliferation of RA FLS in focal inflammation and high expression of CYR61 was studied by primary cell culture. Cultured FLS was stimulated with synovial fluid (SF) of RA patiens, with results showing that SF of RA patients could provoke proliferation of FLS apperantly and up-regulate expression of CYR61 notably. To further research the function of CYR61, we prepared and screened monoclonal antibodies which could combine with natural CYR61 protein. The concentration of CYR61 in RA synovial fluid was detected by ELISA and results showed that CYR61 in high concentration existed in RA SF. To confirm the role of CYR61 in proliferation of FLS, we adopted method of blocking CYR61 with monoclonal antibody to study the effect of CYR61 on FLS proliferation. Results showed that due to blocking CYR61 in SF, proliferation of FLS was significantly reduced, suggesting that CYR61 played an important role in FLS proliferation stimulated by RA SF.
3. The mechanism by which CYR61 regulates FLS proliferation was investigated by siRNA interference. Expression of CYR61 in FLS was knocked down by screened CYR61-specific siRNA and therefore proliferation of FLS was diminished while apoptotic FLS increased, which further confirmed that CYR61 might take effect through inhibiting apoptosis and promoting proliferation of FLS. To determine whether apoptosis family molecules participate in this process, expression profile of Bcl-2 family was detected by real-time PCR before and after CYR61 interference. Results revealed that accompanied with CYR61 inhibited, expression of Bcl-2, inhibitor of apoptosis, was reduced obviously, whereas expression of other genes in Bcl-2 family has no substantial changes. All these results suggest that CYR61 up-regulates Bcl-2 to inhibit apoptosis of FLS and hence promotes proliferation of FLS.

The benefit of this invention lies are:
1. This invention explores application of CYR61 protein for medical purpose, and develops a new application of CYR61 in pharmaceutical field.
2. The results showed that CYR61 could promote proliferation of RA FLS. One of mechanisms is that CYR61 inhibits apoptosis of FLS and up-regulates matrix metalloproteinases (MMPs) to participate in destruction of cartilage and bone. These results provides experimental basis for search biological and small molecular targeting for treatment. These indicate that CYR61 may play very important value in clinical treatment as implicated target.

### Figure legend

Fig1. Relative expression of CYR61 mRNA in synovial tissue (ST) and in fibroblast-like synoviocytes (FLS) from patients with rheumatoid arthritis (P < 0.0001***; P=0.0054 **)
Fig 2. H&E staining and immunohistochemical analysis of synovial tissue
   A. H&E staining for normal synovial tissue (20×)
   B. H&E staining for osteoarthritis synovial tissue (20×)
   C. H&E staining for rheumatoid arthritis synovial tissue (20×)
   D. Immunohistochemical analysis of CYR61 for normal synovial tissue (40×)
   E. Immunohistochemical analysis of CYR61 for osteoarthritis synovial tissue (40×)
   F. Immunohistochemical analysis of CYR61 for rheumatoid arthritis synovial tissue (40×)

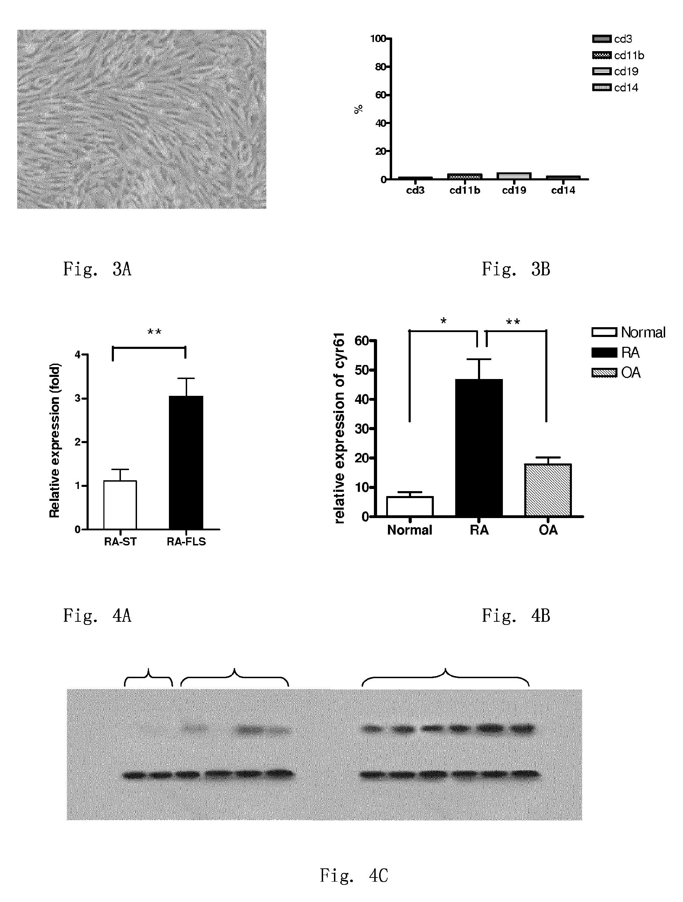

Fig 3A. Culture and identification of FLS in vitro, the third passage (400×)
Fig 3B. Culture and identification of FLS in vitro (< 2% CD11b, <2% CD14⁺, <1%CD3⁺, and <5% CD19⁺, identified using flow cytometer). Expression of CYR61 in synovial tissue (ST) and in fibroblast-like synoviocytes (FLS) from patients with rheumatoid arthritis.
Fig 4A. High expression of CYR61 in RA FLS cultured in vitro, the expression of CYR61 in RA FLS is obviously higher than that in RA synovial tissue.
Fig 4B. High expression of CYR61 in RA FLS cultured in vitro, the relative expression of CYR61 mRNA in RA FLS is obviously higher than that in FLS from OA and normal patients.
Fig 4C. High expression of CYR61 in RA FLS cultured in vitro.
   Western blot: expression of CYR61 protein of FLS from RA, OA and normal patients.

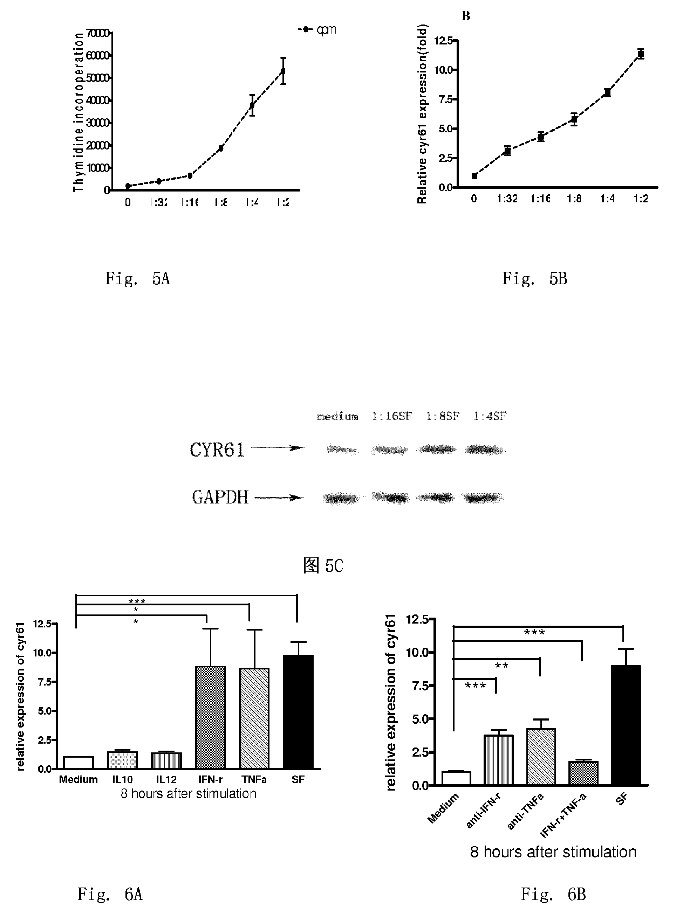

Fig 5A. Influence on FLS proliferation by different concentrations of synovium.
Fig 5B. Influence on expression of CYR61 mRNA of FLS by different concentrations of synovium.
Fig 5C. Influence on expression of CYR61 protein of FLS by different concentrations of synovium.
Fig 6A. Increased expression of CYR61 in RA FLS regulated by IFN-γ and TNF-α.
   Expression of CYR61 in RA FLS stimulated by different cytokines(concentration: IL-10 0.1ng/ml, IL-12 0.5ng/ml, IFN-γ 0.05ng/ml, TNF-α 0.05ng/ml)
Fig 6B. Increased expression of CYR61 regulated by IFN-γ and TNF-α of RA FLS.
   Influence on IFN-γ, TNF-α and SF stimulation effect by blocking antibody(anti-IFN-γ (10µg/ml) and anti-TNF-α)

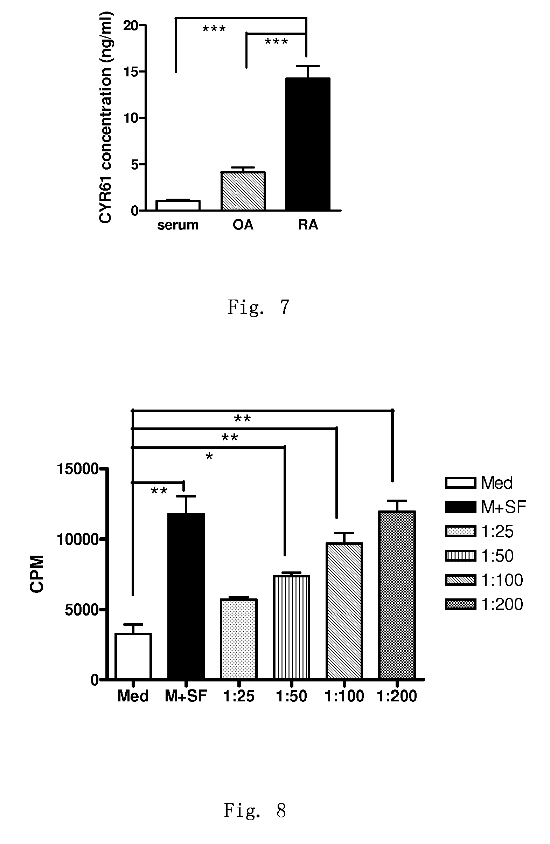

Fig 7. Measurement of CYR61 in RA and OA SF and RA serum by ELISA.
   Levels of CYR61 in RA SF were higher than that in OA SF or in RA serum.
Fig 8. FLS proliferation stimulated by CYR61 in RA SF
   CYR61 specific monoclonal antibody with dilutions of SF (1:25, 1:50, 1:100, 1:200) were added into FLS (See the X axis under the label). Unrelated antibody was added into the M+SF group (black bar);FLS proliferation was detected by H3-TdR.

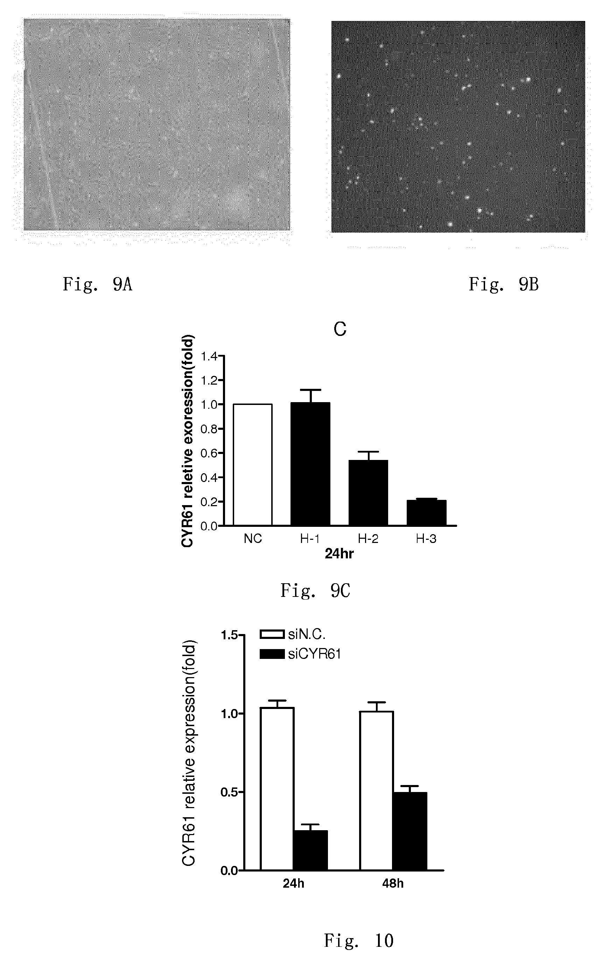

Fig 9A. Screening for CYR61 gene-specific siRNA
   Observe the transfection efficiency of liposomes through watching the condition of transfected fluorescently labeled small RNA into cells using optical microscope.
Fig 9B. Screening for CYR61 gene-specific siRNA
   Observe the transfection efficiency of liposomes through watching the condition of transfected fluorescently labeled small RNA into cells using fluorescence microscope.
Fig 9C. Screening for CYR61 gene-specific siRNA
   CYR61 gene-specific siRNA was transfected into human 293T cell line for 24h, then the expression of CYR61 mRNA was detected by realtime PCR, and the third one(H-3) is the most efficient one.
Fig 10. Phase analysis of CYR61 gene-specific siRNA
   CYR61 gene-specific siRNA was transfected into RA FLS using for 24h, then the expression of CYR61 mRNA was detected by realtime PCR.

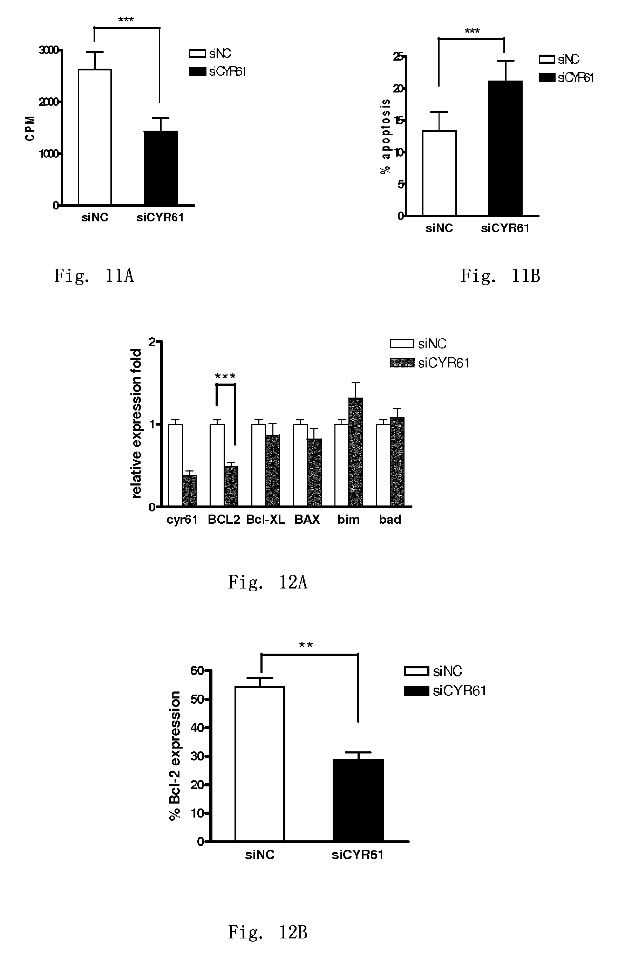

Fig 11A. Decreased proliferation of RA FLS by CYR61 gene knockdown
Fig 11B. Increased apoptosis of RA FLS by CYR61 gene knockdown
Fig 12B. Expression of members of bcl-2 gene family in RA FLS after CYR61 gene knockdown
Fig 12B. Expression of bc1-2 protein in RA FLS after CYR61 gene knockdown Specific implementation plan

The following we will do the detailed illustration combination of figures toward specific implementation plan of the application of CYR61 protein provided by our invention in the pharmacy.

### Example 1:

Expression of CYR61 in synovial tissue (ST) from patients with rheumatoid arthritis

### I. Materials and Methods

### 1. Experimental materials

### 1.1 Clinical specimens

All samples in the research were obtained from clinical orthopaedic knee arthroplasty or synovectomy of RA and OA patients. The diagnosis accord the international diagnostic criteria. (The diagnosis of RA fulfilled the revised criteria by the American College of Rheumatology and the osteoarthritis (OA) patients fulfilled the diagnosis criteria of OA proposed by Altman).2 trauma patients with no history of acute or chronic arthritis served as controls. FLS obtained from Synovial tissues of patients were cultured in vitro, some of them were fixed in 4% paraformaldehyde and used for immunohistochemistry. Sera and SF specimens were high speed centrifuged, and supernatants were collected and immediately stored at -80°C before use. All consent forms were approved by the Institutional Medical Ethics Review Board of the Shanghai Jiao Tong University School of Medicine.

### 2. Experiment method

### 2.1 Cells preparation

### 2.1.1 Preparation of mononuclear cells from PBMC

PBMC anticoagulated by heparin were separated by density gradient centrifugation using Ficoll-Hypaque. Mononuclear cells were washed twice by phosphate buffered saline (PBS), and then stored for subsequent use.

### 2.1.2 Preparation of mononuclear cell from SF

Synovial fluid anticoagulated by heparin were separated by density gradient centrifugation using Ficoll-Hypaque. Mononuclear cells were washed by phosphate buffered saline (PBS) twice, and then stored for subsequent use.

### 2.2 Preparation and primary culture of FLS

ST specimens were minced into small pieces (1mm*1mm*1mm) and incubated for 2 hours with 1mg/ml type I collagenase in Dulbecco's modified Eagle's medium (DMEM) at 37°C. Cells were collected by filtering the suspension through nylonmesh (70um), and extensively washed and cultured in complete high-glucose DMEM supplemented with 10% fetal calf serum , 2 mM L-glutamine, 100 units/ml penicillin, and 100ug/ml streptomycin in a humidified 5% CO₂ incubator. When the flakiness of FLS>80%, digest and subculture. FLS at passage 3 were staining of CD14, CD11b, CD3, and CD19 surface markers, as identified using a FACS Calibur flow cytometer.

### 2.3 Real-time PCR:

Primers were developed using Primer Express 2.0 software (Applied Biosystems), CYR61 and GADPH (as control) primer sequence is as follows:

**Table 1. Real-time PCR primer sequence**

| Gene | Sequence 5' → 3' |
|---|---|
| GAPDH | ACCCACTCCTCCACCTTTGA (forward) (Seq ID NO.1) |
| | CTGTTGCTGTAGCCAAATTCGT (reverse) (Seq ID NO.2) |
| CYR61 | TCCAGCCCAACTGTAAACATCA (forward) (Seq ID NO.3) |
| | GGACACAGAGGAATGCAGCC (reverse) (Seq ID NO.4) |

| | |
|---|---|
| Total RNA was extracted from specimens using an RNeasy Mini kit (Qiagen). Messenger RNA (mRNA) was converted to cDNA using a Sensiscript RT kit (Qiagen). Two-step real-time PCR was performed using SYBR Green Master Mix (Applied Biosystems) according to the manufacturer's instructions. | |

### 2.4 H&E staining and immunohistochemical analysis for synovial samples

Prepare synovium specimens in accordance with the conventional biopsy requires, dehydrated, fixed, embedded, sliced, and stained. Analyse synovial specimens using HE staining and CYR61-specific immunohistochemical staining.

### 2.5 Graphical analysis of immunohistochemical analysis for synovial samples

After input the image to the image analyzer, remove all kinds of noise and distortion, measurement parameters obtained by the image binarization processing will be converted to Excel for analysis, to acquire the relative amount of protein.

### 2.6 Expression of CYR61 protein evaluated by Western blot analysis in FLS

Tissue or cell lysates was separated by SDS-PAGE electrophoresis followed by transfer to polyvinylidene fluoride membranes. The expression of CYR61 was analyzed using specific Abs. After washing with PBS, the membranes were incubated with HRP-conjugated goat anti-mouse IgG at room temperature for 1 h followed by washing with PBS. The target proteins were examined with an ECL system and visualized with autoradiography film.

### Results:

### 1. High expression of CYR61 mRNA in ST from RA patients analyzed by microarray.

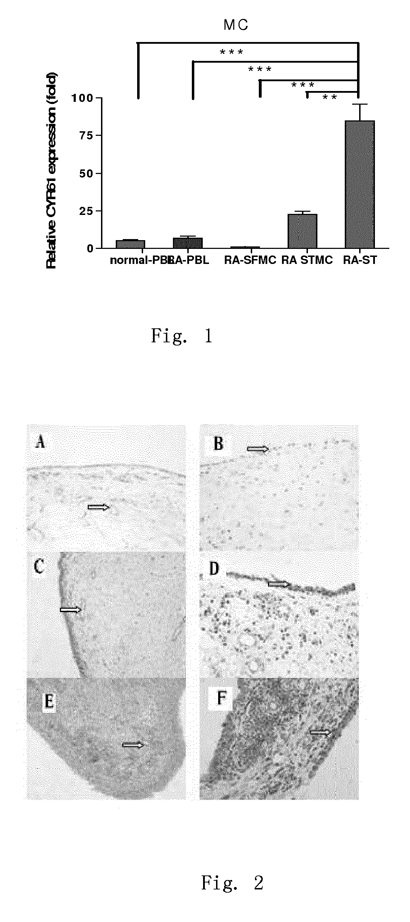

In a preliminary microarray analysis, we observed that CYR61 was high expressed in RA ST. To confirm this result, we examined levels of CYR61 mRNA in isolated STMCs, SFMCs, and PBMCs, as well as in total ST obtained from RA patients (n =10), OA patients (n =10) using real-time PCR.CYR61 and internal reference GAPDH all obtained specific S-shaped amplification curve, and each curve of wells was unanimous, which showed results were reliable (Fig 1). The consequence from real-time PCR showed that expression of CYR61 in RA ST was higher than in STMCs, SFMCs, and PBMCs from RA patients by 8.7 times, 125 times and 13.3 times respectively (p <0.05), confirmed the consequence of microarray.

### 2. High expression of CYR61 mRNA in ST from RA patients analyzed by immunohistochemistry

In order to further validate the results of the above detection by means of molecular biology, we further observed the CYR61 distribution in different cells of synovial tissue and protein expression levels by H&E staining and immunohistochemistry. The results showed that, compared with OA and normal synovial tissue (The arrows indicate no perivascular infiltration of inflammatory cells, Fig2A, 2B), synovium from RA patients could find macrophage-like synovial cell infiltration in synovial lining layer and sublining, vascular proliferation, a large number of lymphocytes, plasma cells and mononuclear cell infiltration, lymphoid follicle formation with fibrinoid necrosis, proliferation of FLS (The arrows indicate perivascular infiltration of inflammatory cells, Fig2C).

On the basis of HE staining, utilizing of immunohistochemical techniques, the synovial tissue of RA patients with the surgical removal was examined for serial sections and ABC staining, finding the expression of CYR61 protein in both the synovial lining cells and sublining vascular endothelial cells, and FLS in rheumatoid arthritis patients (The arrows indicate CYR61 protein as brown particles, Fig 2F) , mainly distributed in the cytoplasm, particularly prevalent in near the joint cavity side of the synovial villi, while OA and normal synovial tissue is rare CYR61 protein expression (The arrows indicate CYR61 protein as brown particles, Fig 2D,2E).

### 3. Identification and culture of FLS derived from RA patients in vitro.

To confirm the result of immunohistochemistry, we established in vitro culture system of FLS.

RA ,OA or normal ST specimens were minced into small pieces and incubated for 2 hours with 1mg/ml type I collagenase in Dulbecco's modified Eagle's medium (DMEM) at 37°C. When the flakiness of FLS>80%, digest and subculture. FLS at passages 3 - 5 growth rapidly and cell morphology tend to be long spindle, arranged in neat rows, consistent orientation (Fig 3A).

Applying specific lymphocytes differentiation antigen staining such as CD3, CD14, CD 19 and CD11C surface markers, identified by flow cytometer, we find all these molecules were negative expression(< 2% CD1Ib, <2% CD14+, <1%CD3+, and <5% CD19+),indicated that the homogeneity of the cultured cells with few lymphocytes pollution(Fig 3B).

### 4. High expression of CYR61 mRNA in FLS from RA patients analyzed by real-time PCR.

To confirm the expression of CYR61 of RA patients, we measured synovial tissue and FLS cultured in vitro using real-time PCR. Expression of CYR61 mRNA was higher in RA FLS than in RA ST (p <0.01) (Figure 4A), indicated the expression of CYR61 was prominent in RA FLS. Simultaneously, we compared CYR61 mRNA expressed by passage 3 FLS between RA, OA and normal samples. The expression of CYR61 mRNA were significantly higher in RA FLS than in OA FLS or in FLS from normal patients respectively by 3.5 times, 20 times (p <0.05)(Figure 4B), proved the increased expression of CYR61 in RA FLS in mRNA level.

Furthermore, we tested CYR61 protein expression in RA FLS, OA FLS or in FLS from normal patients by Western-blot. FLS from RA, OA and normal patients added with lysis Buffer were separated by 12%SDS-PAGE electrophoresis followed by transfer to polyvinylidene fluoride membranes. The expression of CYR61 was stained with rabbit anti-CYR61 polyclonal antibody (1:200 dilution) incubated overnight. After washing with PBS, the membranes were incubated with HRP-conjugated goat anti-mouse IgG (1:2000 dilution) at room temperature for 1 h followed by washing with PBS. The target proteins were examined with an ECL system and visualized with autoradiography film. These results suggest that expression level of CYR61 protein was significantly higher in RA FLS than in OA FLS or in FLS from normal patients, consequently revealed that expression of the CYR61 protein was prominent in RA FLS.

The establishment of such a system laid the foundation for further analysis the biological functions of CYR61 in FLS growth and RA synovial inflammation.

### Example 2: FLS proliferation stimulated by CYR61 in RA SF

### Materials and Methods

### 1.1 Synovial fluid stimulation experiment:

3-5 samples of synovial fluid mixed together were added into FLS at the log phase and cultured for 24h. Detect the expression levels of both CYR61 mRNA and the CYR61 protein, or FLS proliferation. (Described in the following)

### 1.2 FLS proliferation and antibody neutralization assays

RA FLS were cultured for 24 hours at a density of 1×10⁴cells/ml in 96-well plates in complete DMEM in the presence of diluted RA SF (1:2, 1:4, 1:8, 1:16, 1:32) or cytokines (IL-10, IL12, IFNγ, TNFa and IL-17) or CYR61 (5-20 ug/ml). FLS were pulsed with 1µCi thymidine for an additional 16 hours, and incorporated radioactivity was measured as counts per minute using a beta-plate counter.
For the antibody blocking assay, mouse anti-CYR61 mAb (1 mg/ml,diluted 1:25-1:200) was preincubated for 2 hour before stimulation of FLS proliferation. An isotype-matched antibody was used as a control. The rest of the operation was same with the former.

### 1.3 Concentrations of CYR61 from SF and PBMC measured using a sandwich ELISA.

Collect 30 samples of RA and OA SF and PBMC from normal individuals (as control) respectively, and store in -80°C. When detection, microtiter plates were coated with rabbit anti-human CYR61 (Purchase the standard anti-CYR61 antibody as a correction and positive control) and stored overnight at 4°C. SF or serum along with the standard were diluted with phosphate buffered saline (PBS) and added to duplicate wells. Plates were incubated at 37°C for 2 hours and subsequently washed with PBS-Tween 20. An IgG1 mouse anti-human CYR61 mAb (prepared in our laboratory) was added and incubated at 37°C for 2 additional hours. After washing, horseradish peroxidase-conjugated goat anti-mouse IgG antibodies and, later, tetramethylbenzidine were used for color development. Concentrations of CYR61 were evaluated according to absorbance at 492nm using Microplate software.

### 1.4 Preparation of anti-human CYR61 monoclonal antibody (mAb):

CYR61 protein expressed by gene recombination technology emulsified with equal volume of CFA and immunized mice subcutaneously. The spleen cells removed from immunized mouse were fusioned with myeloma cells SP2 / 0. Limiting dilution and screened the specificity cell clones which could bind genetically engineered protein CYR61. The supernatant obtained was determined by enzyme-linked immunosorbent assay (ELISA). The NO.3 monoclonal antibody was obtained on June 22, 2006, another one was obtained on December 7, 2007.

### 1.5 Preparing and purification of Anti-CYR61 monoclonal antibody from ascitic fluid

CYR61 hybridoma cells were injected into 6 to 8 weeks female BALB / c mice through intraperitoneal side. When the enterocoelia of mice were obviously swelling after 8 to 10 days, packed sterile ascitic fluid stored at -80 ° C.

### Results

**1. SF derived from RA patients promote FLS proliferation and CYR61 expression.** To observe the effects Local inflammatory environment may has on the biological effect and CYR61 proliferation level of FLS, we added SF derived from RA patients into FLS culturing and observed FLS proliferation under stimulation with different concentration SF by H3-TdR incorporation. As is shown in figure 5A, SF promoted the FLS proliferation indicated by increased synthesis of DNA in RA FLS in a dose-dependent manner. Comparing with the control group, each dose group of SF obviously promoted the synthesis of DNA in RA FLS (P<0.01). *In vitro* assay, different concentrations SF from RA patients were used to accumulate FLS obtained from RA patients. We found that the more SF were added into FLS culturing, the more expression of CYR61 in FLS. These results confirmed that the FLS proliferation and CYR61 expression with SF-added dose dependent manner. We also found that the stimulus disappeared when RA SF was diluted to 1:32. We examined the CYR61 mRNA and protein by real-time PCR (Figure 5B) and western-blot (Figure 5C) respectively.
2. **IFN-γ and TNF-α in RASF can up-regulate CYR61 expression.** It is well known that SF contains high concentration of cytokines. In order to clarify whether these cytokines relate to up-regulation of CYR61, we stimulated RA FLS by purified cytokines in the dose-detected in SF, and observed the expression profile of CYR61. The results showed that: both IFN- y and TNF- α showed up-regulating CYR61 expression. Premixed anti-IFN-γ antibody (10µg/ml) and anti-TNF-α antibody (200ng/ml) with SF blocked FLS proliferation obviously. This result cleared these two cytokines play critical role in promoting FLS proliferation by up-regulating CYR61. During this study, CYR61 expression in FLS was detected using real-time PCR from cultured cells which have been stimulated by IFN-γ and TNF-α or blocking antibodies mixed with SF for 8 hours. The results showed that both IFN-γ and TNF-α could up-regulate CYR61 expression respectively. Furthermore, the stimulation was stronger as SF when mixed IFN-γ and TNF-α was added into FLS culturing (Figure 6A). These indicated that both IFN-γ and TNF-α could promote FLS proliferation by up-regulate CYR61 expression as SF does (Figure 6B).
3. **There is high level of CYR61 in.** CYR61, a secreted protein, which has mitogenic activity, can induce fibroblasts' proliferation. In addition, we found that over expression of CYR61 FLS derived from RA. To confirm this finding, we prepared hCYR61 neutralization monoclonal antibody, mAb3 (see 2.8, preparation of mAb) and detected the concentration of CYR61 in RA SF (n=30). The results showed that the level of CYR61 in RA SF was significantly higher than that of in OA SF or in RA serum (P<0.0001, Figure 7).
4. **FLS proliferation stimulated by CYR61 in RA SF.** To study whether CYR61 can stimulate FLS proliferation in RA, we used mAb3 for neutralizing CYR61 in RASF and examined mAb3 inhibition on FLS proliferation stimulated by CYR61. FLS of logarithmic growth phase were seeded in a 96-well plates in presence of mixed SF with mAb3 with a dilution of 1:25, 1:50, 1:100 and 1:200. A non-related mAb (1:25) was as control. Thymidine incorporation assay were used to detect FLS proliferation promoted by SF. The results showed that mAb3 which is against human CYR61 could inhibit the FLS proliferation stimulated by RA SF in a dose-dependent manner. When mAb3 was diluted to 1:200, the blocking effect of CYR61 disappeared (Figure 8). Thus indicated that CYR61 stimulate FLS proliferation in RA SF.
Examples 3: mechanism of CYR61 promoting proliferation of FLS derived from patients with rheumatoid arthritis

### Materials and methods

**1. Clinical samples, gene expression and protein detection of CYR61 are the same as the materials and methods of "example 1: expression pattern of CYR61 in RAST".**
**2. FLS culture and proliferation Assay is the same as the materials and methods of "example 2: CYR61 in RASF promote the proliferation of FLS".**
3.1 **SiRNA sequence and synthesis**. 3 SiRNAs were synthesized according to SiRNA design principle. As follows:

| | |
|---|---|
| S1: | 5'-CCA GAAAUG UAU UGU UCAATT-3- (Seq ID NO.5) |
| | 5'-UUG AAC AAU ACA UUU CUG GCC-3- (Seq ID NO.6) |
| S2: | 5'-UCAAGG UAU CAA UGU UUAATT-3- (Seq ID NO.7) |
| | 5'-UUG CUC GCAACAAAU CUG CTC -3- (Seq ID NO.8) |
| S3: | 5'-CAA CGA GGA CUG CAG CAAATT-3- (Seq ID NO.9) |
| | 5'-UUU GCU GCA GUC CUC GUU GAG -3- (Seq ID NO.10) |

### 3.2 SiRNA transfection assay:

Transfection reagent liposomes (geneporter) and DMEM solution containing antibiotics without serum were used in a 250µl transfection system (24-well plates as an example). 5X10⁴ of 3-5 generation FLS were seeded in 24-well plates and incubated at 37°C, 5%CO₂ overnight. 2µl SiRNA was add to a mixture containing 12µl geneporter and 113µl DMEM, staying at room temperature for 25minutes. Then we added this blended transfection mixture to previously incubated FLS (nonrelated SiRNA as control), 37°C, 5 % CO₂ for 5-7hours, then equal volume of DMEM supplemented by 20% FBS and antibiotics were added and incubated continuously for another 24-48 hours. The inhibition effects of SiRNA were detected by real-time PCR and Western blot on the final incubated FLS.

4.0 **Study on CYR61 inhibition of FLS apoptosis.** We divided the previous CYR61-RNA-interfered FLS into 3 groups: 100nM NC transfection siRNA, 100nM S3 transfection siRNA and normal cells group. These FLS transfected SiRNA was collected in 24 hours. The proliferation and apoptotic cells were detected. The apoptosis-related genes (Bc1-2, Bc1-x1, Bax, Bad, Bim) were examined.

### II. Result

**1. CYR61 specific small RNA screening.** We designed 3 kinds of double-stranded small RNA (siRNA) against human CYR61 gene. Mammalian unrelated siRNA was used as negative control (siRNA of negative control, siNC). We transfected siRNA by liposomes (geneporter) into FLS. We identified the transfected FLS by optical microscope (Figure 9A) and fluorescence microscopy (Figure 9B) for identified FLS with labeled siRNA and we found that the transfection rate reached to 80%-90% FLS. The expression of CYR61 after interferon with SiRNA was detected at 24 hours by real-time PCR. Our results showed that among the 3 kinds of double-stranded small RNA, H-3 could inhibit human CYR61 expression specifically and its suppression rate is proximately 70%-80% (Figure 9C).
2.**Analysis of kinetic expression of human CYR61 by siRNA interference.** CYR61 specific siRNA was used to block the expression of CYR61 in FLS from RA, and non-related siRNA as control. Real-time PCR was applied to detect the expression of CYR61 at different interference time. The results showed that only the 20%-30% normal level (e.g. co-cultured with non-relative SiRNA) CYR61 expressed in FLS in presence of SiRNA against hCYR61 in 24h, while expression of CYR61 increased to 40% normal level of CYR61 after 48h in presence of SiRNA (p<0.05, Figure 10). These indicated that SiRNA against CYR61 inhibited expression of CYR61 significantly after 24h transfected into FLS.
3. **Decreased proliferation and increased apoptosis of FLS after CYR61-specific siRNA interfering.** Previous research has shown that SF stimulated FLS proliferation in a dose-dependent manner, which can be blocked by CYR61 neutralizing antibody. These results reveal that CYR61 plays an important role in FLS proliferation. Further, to identify the relationship between CYR61 and FLS proliferation, specific siRNA was applied to block CYR61 gene in RA FLS with unrelated siRNA as control. ³H incorporation assays were used to test the stimulated proliferation of FLS in RA. The proliferation of FLS was declined due to suppressed CYR61 (p<0.0001, Figure 11A). To probe into the correlation between CYR61 and proliferation of FLS in RA, FACS (Annexin-V+PI) was used to analyze the apoptotic RA FLS interfered by siRNA. The result showed that percent of apoptotic FLS with specific SiRNA interfering increased markedly (p<0.0001, Figure 11B).
4. **Mechanism of increased apoptosis of FLS by CYR61-specific siRNA interference**. The result that increased apoptosis of RA FLS interfered by CYR61-specific siRNA clarified the role of CYR61 in this process. To deeply explore the relationship between CYR61 and apoptosis-related genes, siRNA is applied to interfere expression of CYR61 in FLS *in vitro* and the suppressive rate is more than 60%. Mitochondrion apoptosis pathway-related genes bcl-2, bcl-xl, bax, bim and bad were tested by real-time PCR, and the result indicate that the mRNA level of suppressive apoptosis-related gene Bcl-2 was declined (p<0.001, Figure 12A) while Bim, apoptosis enhancing gene, increased slightly. Meanwhile, Bad, Bax and Bcl-xl did not change obviously. These results suggested that the effect of CYR61 on FLS proliferation might duo to the decreased expression of Bcl-2, suppressive apoptosis-related gene. We also tested the protein level of Bcl-2 by FACS and found Bcl-2 decreased markedly in FLS (p<0.01, Figure 12B). Thus, CYR61 inhibits FLS apoptosis though suppressing Bcl-2 family molecules expression (mainly through down-regulate protein Bcl-2) which mediated apoptosis by mitochondrion pathway.

The above described is only a preferred embodiment of this invention. The general technical staff in technical field, on the premise of the method of the invention, could make improvements and supplements, which should also be regarded as the scope of the protection of this invention.

## Claims

1. An application of CYR61 protein in preparation of medicine is for rheumatic diseases treatment.

2. According the application described in claim 1, the characteristic of applied rheumatoid diseases is rheumatoid arthritis.

3. Application of CYR61 inhibitor medicine prepared for anti-rheumatoid diseases.

4. According to the application described in claim 3, the rheumatoid disease is rheumatoid arthritis.

5. According to the application described in claim 3 and/or claim 4, the characteristic of described CYR61 inhibitor are anti-CYR61 antibodies, specific CYR61 gene interference RNAs.

6. According to the application described in claim 5, the characteristic of described anti-CYR61 antibodies are monoclonal antibodies.

7. According to the application described in claim 5, the characteristic of described specific CYR61 gene interference RNA are one of siRNAs (listed as S1, S2 and S3) or their isoforms:
S1 siRNA: Sense strand is SEQ ID No.5, Antisense strand is SEQ ID No.6,
S2 siRNA Sense strand is SEQ ID No.7, Antisense strand SEQ ID No.8, and,
S3 siRNA Sense strand is SEQ ID No.9, Antisense strand SEQ ID No.10.

8. According to the application described in claim 5, the characteristic of described specific CYR61 gene interference RNAs (siRNA) are selected from:
S1 siRNA: Sense strand is SEQ ID No.5, Antisense strand is SEQ ID No.6,
S2 siRNA Sense strand is SEQ ID No.7, Antisense strand SEQ ID No.8,and,
S3 siRNA Sense strand is SEQ ID No.9, Antisense strand SEQ ID No.10.

9. Specific CYR61 gene interference RNAs (siRNA) are selected from:
S1 siRNA: Sense strand is SEQ ID No.5, Antisense strand is SEQ ID No.6,
S2 siRNA Sense strand is SEQ ID No.7, Antisense strand SEQ ID No.8, and,
S3 siRNA Sense strand is SEQ ID No.9, Antisense strand SEQ ID No.10.
